# EUROPÄISCHE PATENTANMELDUNG

(11) **EP 2 472 256 A1**
(43) Veröffentlichungstag der Anmeldung: **04.07.2012**
(21) Anmeldenummer: 11195846.8
(22) Anmeldetag: 28.12.2011
(51) Int. Cl.: G01N 33/28

(54) **Vorrichtung und Verfahren zum automatischen Überwachen des Gebrauchszustands eines Kühlschmierstoffs**

(30) Priorität: 30.12.2010 DE 102010056551
(71) Anmelder: Universität Bremen, 28359 Bremen (DE); IWT Stiftung Institut für Werkstofftechnik, 28359 Bremen (DE)
(72) Erfinder: Larek, Roland, 49692 Cappeln (DE); Koch, Thomas, 28199 Bremen (DE); Brinksmeier, Ekkard, 28213 Bremen (DE)
(74) Vertreter: Scholz, Volker

(57) **Zusammenfassung**

Die vorliegende Erfindung betrifft eine Vorrichtung zum automatischen Überwachen des Gebrauchszustands eines Kühlschmierstoffs (KSS), welche umfasst zumindest einen Gassensor, wobei der Gassensor so ausgestaltet ist, dass er eine oder mehrere, im wesentlichen flüchtige Substanz(en), vorzugsweise resultierend aus mikrobiellem Abbau, aus dem Kühlschmierstoff in einem Gasraum detektieren kann, eine Auswerteeinheit in Wirkverbindung mit dem zumindest einen Gassensor, die bei qualitativer Detektion zumindest einer flüchtigen Substanz ein Signal erzeugt sowie ein Verfahren zum automatischen Überwachen des Gebrauchszustands eines Kühlschmierstoffs.

## Beschreibung

Die vorliegende Erfindung betrifft eine Vorrichtung zum automatischen Überwachen des Gebrauchszustands eines Kühlschmierstoffs sowie ein diesbezügliches Verfahren.

Unter einem Kühlschmierstoff (KSS) soll im Rahmen der vorliegenden Erfindung ein Kühlschmierstoff gemäß DIN 51385 verstanden werden. Kühlschmierstoffe dienen in der Fertigungstechnik beim Trennen und Umformen auf Werkzeugmaschinen der Wärmeabfuhr und Verminderung der Reibung zwischen Werkzeug und Werkstück durch Schmierung. Kühlschmierstoffe und Schmierstoffe sind gemäß DIN 51502 deutlich voneinander abgegrenzt.

Gemäß DIN 51385 werden wassermischbare und nicht wassermischbare Kühlschmierstoffe unterschieden. Wassermischbare Kühlschmierstoffe können beispielsweise einen Anteil von bis zu 95 Gewichtsprozent Wasser, bezogen auf das Gesamtgewicht des Kühlschmierstoffs, enthalten. Insbesondere für wassermischbare Kühlschmierstoffe ist es bekannt, dass diese durch Mikroorganismen wie Bakterien und Pilze befallen werden können. Besondere bei seit längerem in Gebrauch befindlichen Kühlschmierstoffen kommt es während längeren Arbeitspausen des Öfteren zu Bakterien- und Pilzbefall, was sich schließlich in starker Geruchsbildung und eventuell auch Verfärbung des Kühlschmierstoffs bemerkbar macht. Geruchsbildung und Verfärbung sind meist jedoch erst dann feststellbar, wenn ein sehr starker Befall durch Mikroorganismen vorliegt. Häufig resultieren aus dem Befall gesundheitliche Beschwerden bei den Arbeitern, welche mit Kühlschmierstoffen in Kontakt kommen. Technische Probleme können auch durch die Verstopfung von Rohrleitungen durch Pilzfäden hervorgerufen werden.

Darüber hinaus sind Kühlschmierstoffe auch hohen mechanischen Belastungen ausgesetzt und unterliegen somit einem zeitlichen Verschleiß. Eine verminderte Qualität von Kühlschmierstoffen kann zu Abweichungen im Produktionsergebnis bis zum Produktionsausfall führen.

Aus diesem Grunde ist es notwendig, die Qualität des eingesetzten Kühlschmierstoffs regelmäßig zu überprüfen.

Im Stand der Technik erfolgt die Beprobung von Kühlschmierstoffen entweder durch physikalisch-chemische Analyseverfahren oder mittels Bestimmung mikrobiologischer Parameter. Letzteres erfolgt in der Regel über die Zellzahlabschätzung mit Hilfe sogenannter Dip-Slides. Ein Nachteil bei diesem Verfahren ist die lange Untersuchungsdauer aufgrund einer Inkubationszeit von mindestens 48 Stunden.

Ein weiteres Verfahren stellt die Bestimmung des Gehalts an Adenosintriphosphat (ATP) dar. Der ATP-Gehalt gibt einen Hinweise auf den Anteil des in allen lebenden Zellen vorkommenden Energielieferanten ATP in der untersuchten Probe und lässt somit einen Rückschluss auf das Vorkommen von Mikroorganismen in der Probe zu, jedoch nicht auf die Zellzahl als solche.

Zu den physikalisch-chemischen Verfahren gehören die Messungen der Kühlschmierstoffkonzentration über refraktometrische Bestimmung sowie die mittels einfacher Teststreifen bestimmbaren Parameter, wie pH-Wert und Nitrat- bzw. Nitrit-Gehalt. Weitere Verfahren zur chemischen Charakterisierung von Kühlschmierstoffen mittels Auftrennungsverfahren sind meist mit einem hohen apparativen Aufwand verbunden.

Nachteilig bei allen aus dem Stand der Technik bekannten Verfahren und Vorrichtungen ist insbesondere, dass eine Probennahme erforderlich ist und üblicherweise die Auswertung der Probe eine längere Zeit in Anspruch nimmt.

Aus der DE 103 27 625 B4 sind eine Vorrichtung und ein Verfahren zur automatischen Überwachung des Gebrauchszustands eines Schmierstoffs gemäß DIN 51502 einer Maschine oder eines Maschinenteils bekannt. Die dort beschriebene Vorrichtung erfordert das Vorhandensein einer Probenkammer sowie einer Sensorkammer, in welchen die zu untersuchende Probe auf physikalische Probenbedingungen (insbesondere Temperatur) eingestellt wird. Die Messung beruht auf der Konzentration eines oder mehrerer flüchtiger Bestandteile in einem angereicherten Trägergas. Die Messung erfolgt somit in einer von der den Schmierstoff einsetzenden Maschine getrennten apparativen Einheit in Abhängigkeit der Konzentration der flüchtigen Bestandteile. Der Einfluss mikrobieller Lasten wird nicht beschrieben.

Die DE 199 47 669 A1 beschreibt ein Verfahren zur Bestimmung der Qualität von Fetten und Ölen sowie eine Vorrichtung zur Durchführung des Verfahrens. Das Messverfahren zeichnet sich dadurch aus, dass mittels eines ersten Gassensors die Konzentration der schwerflüchtigen Kohlenwasserstoffe bestimmt wird, aus der ein Kriterium für die Qualität des Fettes oder Öles abgeleitet wird.

Schließlich beschreibt die DE 102 43 510 A1 eine Vorrichtung zur Zustandsbestimmung von Öl, wobei die Konzentration von flüchtigen Bestandteilen des Öls bestimmt wird.

Der vorliegenden Erfindung liegt die Aufgabe zugrunde, eine Vorrichtung und ein Verfahren zum automatischen Überwachen des Gebrauchszustands- eines Kühlschmierstoffs bereitzustellen, so dass online, also im laufenden Betrieb der den Kühlschmierstoff verwendenden Maschine, eine Qualitätsbestimmung des Kühlschmierstoffs durchgeführt werden kann, ohne dass eine externe und/oder zeitaufwändige Untersuchung einer Probe des Kühlschmierstoffs vorgenommen werden muss.

Die Aufgabe wird gelöst durch eine Vorrichtung zum automatischen Überwachen des Gebrauchszustands eines Kühlschmierstoffs (KSS), welche zumindest einen Gassensor, wobei der Gassensor so ausgestaltet ist, dass er eine oder mehrere flüchtige Substanz(en), resultierend aus mikrobiellem und/oder chemischem Abbau, aus dem Kühlschmierstoff in einem Gasraum detektieren kann, und eine Auswerteeinheit in Wirkverbindung mit dem zumindest einen Gassensor, die bei qualitativer Detektion zumindest einer flüchtige Substanz ein Signal erzeugt, umfasst.

Im Rahmen der vorliegenden Erfindung soll unter einer "flüchtigen" Substanz eine Substanz verstanden werden, die aufgrund ihres Gasdrucks unter normalen Umgebungsbedingungen, die am Ort des Gassensors vorherrschen, in die Gasphase übergeht. Unter normalen Umgebungsbedingungen werden in einem Arbeitsraum einer den Kühlschmierstoff einsetzenden Werkzeugmaschine beispielsweise Beriebsbedingungen verstanden, unter denen die eingesetzten Kühlschmierstoffe üblicherweise nicht verdampfbar sind, mögliche Abbauprodukte derselben jedoch schon.

In einer besonders bevorzugten Ausführungsform der vorliegenden Erfindung detektiert der Gassensor nicht das Vorliegen/Nicht-Vorliegen einer einzelnen flüchtigen Substanz, sondern vielmehr eine Mischung unterschiedlicher und typischer Substanzen bzw. Stoffgruppen, wie Alkohole, Ketone, Fettsäuren oder Ester, welche mikrobiell und/oder chemisch durch Abbau aus dem Kühlschmierstoff entstehen. Dabei kann (können) der (die) Gassensor(en) auf die zu detektierenden Substanzen trainiert werden.

Besonders bevorzugt ist, dass die flüchtige Substanz aus einem Abbau einer dem Kühlschmierstoff zugesetzten Markersubstanz resultiert.

Desweiteren ist bevorzugt vorgesehen, dass der Kühlschmierstoff ein wassermischbarer Kühlschmierstoff ist.

Ferner ist bevorzugt, dass der Gassensor in einem Arbeitsraum einer den Kühlschmierstoff einsetzenden Maschine und/oder in einem Kühlschmierstoffsystem selbst, vorzugsweise einem Kopfraum eines Kühlschmierstofftanks, angeordnet ist.

Besonders bevorzugt ist, dass der zumindest eine Gassensor beheizt ist.

Weiter ist vorgesehen dass die Vorrichtung eine Anzeige- und/oder Bedieneinheit umfasst, vorzugsweise in Wirkverbindung mit der Auswerteinheit.

Ebenfalls bevorzugt ist, dass der Kühlschmierstoff und/oder die Markersubstanz umfasst: Mineralöl auf Naphthenbasis, Mineralöl auf Paraffinbasis, Poly-alpha-Oleime, Poly-iso-buten, Polyglykole, gesättigte Fettsäuren, ungesättigte Fettsäuren, geschwefelte Fettöle, geschwefeltes Poly-iso-buten, Dialkylpolysulfine, Petroleumsulfonate, Alkylphosphate, Mono-, Di- oder Triester der o-Phosphorsäure, Mercaptobenzothiazole, Salze der Naphthensäure, Phenolderivate, N- und/oder O-haltige Heterozyklen, Benzoesäure und deren Derivate, Polyalkylacrylate, Aldehydderivate, Formaldehyd-Abspalter, Carbonsäureester von ein- und mehrwertigen Alkoholen, Carbonsäuren (geradkettig, verzweigt, mehrbasig und/oder ungesättigt), natürliche Fette, aliphatische Amine, Fettamine, aromatische Amine, ein- und mehrwertige Alkohole, Ether mehrwertiger Alkohole und Mischungen derselben.

Zudem ist erfindungsgemäß ein Verfahren zum automatischen Überwachen des Gebrauchszustands eines Kühlschmierstoffs, vorzugsweise im laufenden Betrieb einer den Kühlschmierstoff einsetzenden Maschine, umfassend die Schritte: Detektieren des Vorliegen und/oder Nicht-Vorliegens zumindest einer flüchtigen Substanz, die aus einem mikrobiellen und/oder chemischen Abbau des Kühlschmierstoffs resultiert, in einem Gasraum mit zumindest einem Gassensor, Übermitteln des Detektionsergebnisses des Gassensors an eine Auswerteeinheit, und Erzeugen eines Signals durch die Auswerteeinheit, wenn die flüchtige Substanz qualitativ durch den Gassensor detektiert worden ist.

Die vorliegende Erfindung stellt somit eine Vorrichtung und ein Verfahren bereit, bei denen nach dem Prinzip der elektronischen Nase über den Einsatz von zumindest einem Gassensor die Detektion von volatilen Substanzen ermöglicht wird. Die Gassensoren können dabei ausgewählt werden, um bestimmte Geruchsmuster zu erkennen, wobei eine Besonderheit der Erfindung darin liegt, dass die Gassensoren insbesondere diejenigen volatilen Substanzen mikrobiellen Ursprungs detekieren, die für eine Schädigung des Kühlschmierstoffs charakteristisch sind. Darüber hinaus kann besonders bevorzugt der Kühlschmierstoff mit einer Markersubstanz dotiert werden, um so die Freisetzung einer spezifischen bekannten Substanz zu erreichen. Die Freisetzung dieser Substanz spiegelt dann die Belastung des Kühlschmierstoffs wider.

Die vorliegenden Erfindung weist den Vorteil auf, dass sie im laufenden Betrieb der Kühlschmierstoff einsetzenden Maschine, insbesondere einer Werkzeugmaschine kontinuierlich durchgeführt werden kann und ohne großen apparativen Aufwand innerhalb kurzer Zeit eine Aussage über den Zustand des Kühlschmierstoffs zulässt. Auf diese Weise kann der Austausch des Kühlsehmierstoffs immer zum optimalen Zeitpunkt erfolgen. Daher können Kosten eingespart werden, da der Kühlschmierstoff und damit die Laufzeit der Maschine optimal ausgenutzt werden. Schädigungen bis hin zum Produktionsausfall können vermieden werden.

Die Vorrichtung und das Verfahren gemäß der vorliegenden Erfindung zeichnen sich somit insbesondere dadurch aus, dass sie zur Überwachung des Gebrauchszustands von Kühlschmierstoffen geeignet sind, die sich von im Stand der Technik beschriebenen Schmierstoffen deutlich unterscheiden. Dies gilt insbesondere für wassermischbare Kühlschmierstoffe.

Auch ist ein wesentliches Merkmal der erfindungsgemäßen Vorrichtung und des Verfahrens, dass flüchtige Substanzen detektiert werden, die aus einem mikrobiellen Abbau des Kühlschmierstoffs resultieren. In einer besonders bevorzugten Ausführungsform werden flüchtige Substanzen detektiert, die aus dem Abbau einer Markersubstanz resultieren, die dem Kühlschmierstoff zugesetzt ist. Ferner soll darauf hingewiesen werden, dass erfindungsgemäß keine Konzentrationsmessung der flüchtigen Bestandteile erfolgt, vielmehr lediglich die Gegenwart bzw. Nicht-Gegenwart einer flüchtigen Substanz durch den zumindest einen Gassensor detektiert wird und eine entsprechende Information dann an die Auswerteinheit zur Erzeugung eines Signals weitergeleitet wird.

Die vorliegende Erfindung kann somit auch aufgefasst werden als eine Maschine, vorzugsweise Werkzeugmaschine, die einen Kühlschmierstoff in irgendeiner Weise verwendet, und eine Maschine mit integrierter Vorrichtung zum automatischen Überwachen des Gebrauchszustands des Kühlschmierstoffs.

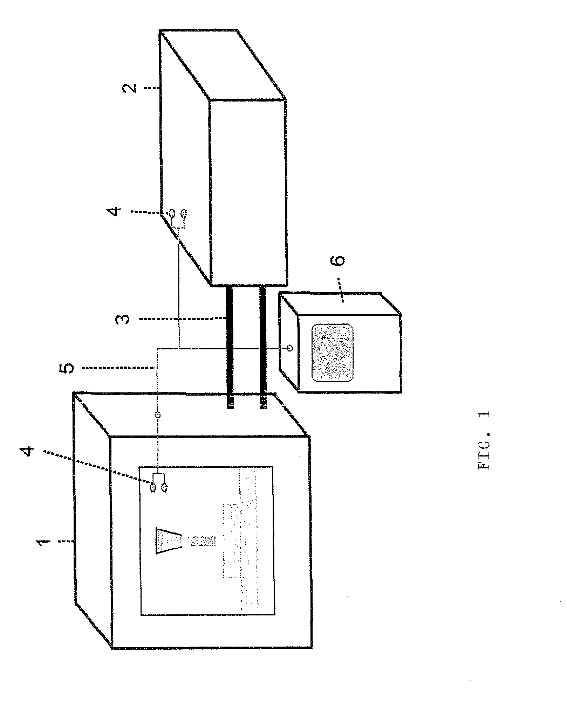

Weitere Merkmale und Vorteile der erfindungsgemäßen Vorrichtung bzw. des erfindungsgemäßen Verfahrens ergeben sich aus der folgenden detaillierten Beschreibung eines bevorzugten Ausführungsbeispiels anhand der beigefügten Zeichnung, in der die Figur einen schematischen Aufbau einer Kühlschmierstoff einsetzenden Maschine mit integrierter Vorrichtung zum automatischen Überwachen gemäß der vorliegenden Erfindung zeigt.

Die Figur zeigt eine erfindungsgemäße Vorrichtung, die in eine Kühlschmierstoff verwendende Maschine 1, vorzugsweise eine Werkzeugmaschine, integriert ist. Die Vorrichtung umfasst ein Kühlschmierstoffsystem mit einem Kühlschmierstofftank 2, der gegebenenfalls mit einer Filteranlage (nicht gezeigt) versehen ist, und über Leitungen 3 zum Transport des Kühlschmierstoffs mit der Maschine 1 verbunden ist. Innerhalb des Kühlschmierstofftanks 2 und/oder im Arbeitsraum der Maschine 1 sind einer oder mehrere Gassensoren 4 angeordnet, die über ein Leitungskabel 5 mit ein Auswerteeinheit 6 verbunden sind. Im Stand der Technik bekannte Gassensoren können verwendet werden. Die Auswerteeinheit 6 kann vorzugsweise zusätzlich ein Anzeige- und/oder Bediengerät umfassen. Die Auswerteeinheit ist bevorzugt ein Computer oder Microcontroller.

Erfindungsgemäß ist auch denkbar, einen Gassensor in einem zentralen Kühlschmierstoffsystem vorzusehen, das mehrere Maschinen 1 versorgt.

Beim kontinuierlichen Betrieb der Maschine 1 wird Kühlschmierstoff aus dem Kühlschmierstofftank 2 über die Leitungen 3 in den Arbeitsraum der Maschine 1 verbracht und aus diesem wieder in den Kühlschmierstofftank 2 zurückgeführt. Insbesondere bei längerem Einsatz des Kühlschmierstoffs kann es zu einem Befall des Kühlschmierstoffs durch Mikroorganismen kommen, was in der Bildung flüchtiger Substanzen resultiert. Der Befall führt schließlich zu einer Schädigung des Kühlschmierstoffs und macht einen Austausch erforderlich.

Das Vorliegen solcher flüchtiger Substanzen mikrobiellen Ursprungs wird durch die Gassensoren 4 detektiert. Bei Detektion einer oder mehrerer solcher Substanzen wird eine Information an die Auswerteeinheit 6 gegeben, die dann ein Signal an eine Bedienperson senden kann.

In einer bevorzugten Ausführungsform ist es vorgesehen, dass die Gassensoren 4 eine oder mehrere flüchtige Substanzen detektieren, die aus einer mikrobiellen Zersetzung einer Markersubstanz resultieren, die dem Kühlschmierstoff zugesetzt worden ist. Dies ermöglicht eine gezielte Einstellung der Vorrichtung bzw. Verfahrens auf ganz bestimmte ausgewählte flüchtige Substanzen, die sich aus der mikrobiellen Umsetzung der Markersubstanz ergeben.

Die Informationen des bzw. der Gassensoren 4 können durch eine spezielle Software ausgewertet und auf einem Display des Bediengeräts zur Anzeige gebracht werden. Über ein Eingabegerät kann der Benutzer ferner mit der Vorrichtung interagieren, um beispielsweise spezielle Aspekte der erfassten Informationen zur Anzeige zu bringen oder Vorrichtungsparameter und/oder Verfahrensparameter zu verändern.

In der oben in Bezug auf die Figur beschriebenen Ausführungsform kann beispielsweise ein Kühlschmierstoff im Wesentlichen auf Basis eines Carbonsäureesters auf Basis eines einwertigen Alkohols, wie beispielsweise Ethanol, eingesetzt werden. Dieser Kühlschmierstoff kann bei mikrobiellem Befall Ethanol durch Spaltung des Esters freisetzen. Das Ethanol ist eine flüchtige Substanz und kann durch geeignete Gassensoren, beispielsweise Sensoren des Typs GGS3000, GGS5000 oder GGS7000 der Firma RST Rostock System-Technik GmbH, detektiert werden. Bei Detektion von Ethanol wird dann durch die Auswerteeinheit in Wirkverbindung mit den Gassensoren ein entsprechendes Signal erzeugt, das anzeigt, dass sich der Gebrauchszustand des Kühlschmierstoffs geändert hat. Die in der vorstehenden Beschreibung, den Ansprüchen und der Zeichnung offenbarten Merkmale der Erfindung können sowohl einzeln als auch in jeder beliebigen Kombination für die Verwirklichung der Erfindung in ihren verschiedenen Ausführungsformen wesentlich sein.

## Patentansprüche

1. Vorrichtung zum automatischen Überwachen des Gebrauchszustands eines Kühlschmierstoffs (KSS), welche umfasst:
- zumindest einen Gassensor, wobei der Gassensor so ausgestaltet ist, dass er eine oder mehrere flüchtige Substanz(en), resultierend aus mikrobiellem und/oder chemischem Abbau, aus dem Kühlschmierstoff detektieren kann, und
- eine Auswerteeinheit in Wirkverbindung mit dem zumindest einen Gassensor, die bei qualitativer Detektion zumindest einer flüchtigen Substanz ein Signal erzeugt.

2. Vorrichtung nach Anspruch 1, **dadurch gekennzeichnet, dass** die flüchtige Substanz aus einem Abbau einer dem Kühlschmierstoff zugesetzten Markersubstanz resultiert.

3. Vorrichtung nach Anspruch 1 oder 2, **dadurch gekennzeichnet, dass** der Kühlschmierstoff ein wassermischbarer Kühlschmierstoff ist.

4. Vorrichtung nach einem der vorangehenden Ansprüche, **dadurch gekennzeichnet, dass** der Gassensor in einem Arbeitsraum einer den Kühlschmierstoff einsetzenden Maschine und/oder in einem Kühlschmierstoffsystem selbst, vorzugsweise einem Kopfraum eines Kühlschmierstofftanks, angeordnet ist.

5. Vorrichtung nach einem der vorangehenden Ansprüche, **dadurch gekennzeichnet, dass** der zumindest eine Gassensor beheizt ist.

6. Vorrichtung nach einem der vorangehenden Ansprüche, umfassend eine Anzeige-und/oder Bedieneinheit, vorzugsweise in Wirkverbindung mit der Auswerteeinheit.

7. Vorrichtung nach einem der vorangehenden Ansprüche, **dadurch gekennzeichnet, dass** der Kühlschmierstoff und/oder die Markersubstanz umfasst: Mineralöl auf Naphthenbasis, Mineralöl auf Paraffinbasis, Poly-alpha-Olefine, Poly-iso-buten, Polyglykole, gesättigte Fettsäuren, ungesättigte Fettsäuren, geschwefelte Fettöle, geschwefeltes Poly-iso-buten, Dialkylpolysulfine, Petroleumsulfonate, Alkylphosphate, Mono-, Di- oder Triester der o-Phosphorsäure, Mercaptobenzothiazole, Salze der Naphthensäure, Phenolderivate, N- und/oder O-haltige Heterozyklen, Benzoesäure und deren Derivate, Polyalkylacrylate, Aldehydderivate, Formaldehyd-Abspalter, Carbonsäureester von ein- und mehrwertigen Alkoholen, Carbonsäuren (geradkettig, verzweigt, mehrbasig und/oder ungesättigt), natürliche Fette, aliphatische Amine, Fettamine, aromatische Amine, ein- und mehrwertige Alkohole, Ether mehrwertiger Alkohole und Mischungen derselben.

8. Verfahren zum automatischen Überwachen des Gebrauchszustands eines Kühlschmierstoffs, vorzugsweise im laufenden Betrieb von den Kühlschmierstoff einsetzenden Maschinen, umfassend die Schritte:
- Detektieren des Vorliegens und/oder Nicht-Vorliegens zumindest einer flüchtigen Substanz aus einem mikrobiellen und/oder chemischem Abbau des Kühlschmierstoffs resultiert, in einem Gasraum mit zumindest einem Gassensor,
- Übermitteln des Detektionsergebnisses des Gassensors an eine Auswerteeinheit, und
- Erzeugen eines Signals durch die Auswerteeinheit, wenn die flüchtige Substanz qualitativ durch den Gassensor detektiert worden ist.
